**Europäisches Patentamt**

(19) **European Patent Office**

**Office européen des brevets**

(11) Veröffentlichungsnummer: **0 024 533**
**B1**

(12) **EUROPÄISCHE PATENTSCHRIFT**

(45) Veröffentlichungstag der Patentschrift:
**29.06.83**

(51) Int. Cl.³: **C 07 D 233/58**

(21) Anmeldenummer: **80104185.6**

(22) Anmeldetag: **17.07.80**

(54) **Isolierung von Imidazolen aus ihren wässrigen Lösungen.**

(30) Priorität: **13.08.79 DE 2932712**

(43) Veröffentlichungstag der Anmeldung:
**11.03.81 Patentblatt 81/10**

(45) Bekanntmachung des Hinweises auf die Patenterteilung:
**29.06.83 Patentblatt 83/26**

(84) Benannte Vertragsstaaten:
**BE CH DE FR GB IT LI**

(56) Entgegenhaltungen:
**DE-A-1 670 459**
**DE-A-2 360 175**
**DE-A-2 614 412**

**Lehrbuch der organischen Chemie, Karrer (1948) S. 92 Handbook of Chemistry and Physics, 45th edition (1973–1974), S. C221 and C413**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hupfer, Leopold, Dr., Waltershoehe 3, D-6701 Friedelsheim (DE)**
Erfinder: **Graf, Fritz, Dr., Im Rothschild 33, D-6720 Speyer (DE)**
Erfinder: **Eberhard, Peter, Dr., Ungsteiner Strasse 2, D-6700 Ludwigshafen (DE)**
Erfinder: **Wickenhaeuser, Gerhard, Dr., Roemerweg 8, D-6701 Birkenheide (DE)**
Erfinder: **Schlueter, Lothar, Dr., Elbinger Strasse 4A, D-7500 Karlsruhe (DE)**

## Isolierung von Imidazolen aus ihren wässrigen Lösungen

Diese Erfindung betrifft ein Verfahren zur Gewinnung von Imidazolen aus ihren wässrigen Lösungen durch Extraktion.

Imidazole lassen sich durch Kondensation von Glyoxal mit Ammoniak und Aldehyden herstellen. Bei dieser bekannten Synthese (M.R. Grimmett, Adv. Heterocycl. Chemistry, Bd. 12 (1970), 103–183) fallen gewöhnlich verdünnte wässrige Imidazollösungen an, die Verunreinigungen und Nebenprodukte enthalten. Diese wässrigen verdünnten Lösungen werden bei Normaldruck oder im Vakuum eingeengt, und die Imidazole aus den so erhaltenen Konzentraten im Vakuum bei hohen Sumpftemperaturen ausgetrieben. Da die so gewonnenen Rohimidazole noch Säuren enthalten, müssen sie häufig noch mit Laugen versetzt und einer zweiten Vakuumdestillation unterworfen werden. Die mit diesem Verfahren verbundenen Nachteile sind offenkundig. Es müssen grosse Mengen Wasser abdestilliert werden, wodurch sich ein hoher Energiebedarf ergibt. Durch das längere Erhitzen der Imidazole in der wässrigen Lösung während des Eindampfvorganges werden ausbeutemindernde Nebenprodukte gebildet. Stark ausbeutemindernd wirkt sich auch die hohe thermische Belastung während der Rohdestillation aus. Zeitraubend und unwirtschaftlich ist ferner die erforderliche zusätzliche Reindestillation unter Laugenzusatz.

In der DE-A-1 670 459 wird ein Verfahren beschrieben, bei dem man Imidazole aus wässrigen Lösungen, die bei der Kondensation von Oxazolen mit Ammoniak oder primären Aminen erhalten werden, durch Extraktion mit Butanol als Lösungsmittel und Destillation der Lösungsmittelphase gewinnt. Bei diesem bekannten Verfahren ist es zur Gewinnung der Imidazole in guten Ausbeuten und reiner Form erforderlich, zu den wässrigen Lösungen vor oder bei der Extraktion alkalisch wirkende Mittel, wie Kaliumcarbonat, zu geben. Auch dieses Verfahren hat somit den Nachteil, dass Laugen zugegeben werden müssen und wässrige Ablaugen mit einer erheblichen Salzfracht anfallen.

Es wurde nun gefunden, dass sich diese Nachteile bei der Gewinnung von Imidazolen aus ihren wässrigen Lösungen, die man durch Kondensation von Glyoxal mit Aldehyden und Ammoniak erhält, durch Extraktion mit einem Lösungsmittel und Destillation der Lösungsmittelphase vermeiden lassen, wenn man als Lösungsmittel mit Wasser nicht mischbare aliphatische Alkohole mit 4 bis 10 C-Atomen verwendet.

Nach dem neuen Verfahren werden die Imidazole in guter Ausbeute und hoher Reinheit gewonnen. Dieses vorteilhafte Ergebnis ist überraschend, da eine so glatte Abtrennung der sehr gut wasserlöslichen Imidazole von den störenden Nebenprodukten mit ähnlichem Lösungsverhalten nicht erwartet werden konnte. Nicht voraussehbar war ferner, dass die als Verunreinigungen enthaltenen organischen Säuren, deren Abtrennung bei der herkömmlichen Gewinnung von reinem Imidazol eine zweite Destillation in Gegenwart von Laugen erforderlich macht, bei dem erfindungsgemässen Verfahren so weitgehend in der wässrigen Phase verbleiben, so dass auf eine gesonderte Destillation verzichtet werden kann. Schliesslich ist auch überraschend, dass sich das vorteilhafte Ergebnis z.B. mit anderen gebräuchlichen Lösungsmitteln, die mit Wasser nicht mischbar sind und die man schon für die Extraktion von Imidazolen aus stark alkalischen wässrigen Lösungen herangezogen hat, wie Methylenchlorid oder Toluol, wegen ihrer unzureichenden Selektivität nicht erzielen lässt.

Nach dem Verfahren der Erfindung werden als Lösungsmittel mit Wasser nicht mischbare aliphatische Alkohole mit 4 bis 10 C-Atomen verwendet, von denen n-Butanol, iso-Butanol, Pentanol und 2-Äthylhexanol bevorzugt sind.

Als Imidazole enthaltende wässrige Lösungen der genannten Art kommen solche in Betracht, die man bei der an sich bekannten Imidazol-Synthese durch Umsetzung von Glyoxal mit Formaldehyd und Ammoniak erhält. Diese wässrigen Lösungen haben einen Imidazol-Gehalt bis etwa 20 Gew.-%. Sie enthalten ausserdem etwa bis zu 5 Gew.-% Ammoniumsalze organischer Säuren, bis zu 5 Gew.-% Ammoniak sowie bis zu 7 Gew.-% andere Kondensationsprodukte und Polymere. Ihr Gehalt an anorganischen Salzen liegt unter 1 Gew.-%. Anstelle des nicht weiter substituierten Imidazols können die wässrigen Ausgangslösungen auch substituierte Imidazole, die z.B. auf entsprechende Weisen synthetisiert wurden, enthalten. Beispielsweise seien folgende Imidazole genannt:

2-Methylimidazol, 4-Methylimidazol,
2-Isopropylimidazol, 2-Äthylimidazol und
2,4,5-Trimethylimidazol.

Man nimmt die Extraktion der wässrigen Imidazollösung vorteilhaft im Gegenstrom bei Raumtemperatur vor. Die Lösungsmittelmenge beträgt dabei etwa die 0,5- bis 5fache Menge, bezogen auf wässrige Imidazollösungen. Die durch die Extraktion erhaltene Lösungsmittelphase wird anschliessend destilliert, wobei das Imidazol vom Lösungsmittel, das erneut zur Extraktion herangezogen werden kann, und vom Rückstand abgetrennt wird. Man erhält dabei die Imidazole in hoher Reinheit. Die Imidazolverluste betragen bei diesem Verfahren weniger als 1%, während sie bei der oben erwähnten bekannten Aufarbeitung der wässrigen Imidazollösungen durch Eindampfen und Destillation 20% erreichen.

Beispiel

Von einer wässrigen Rohimidazollösung, die auf an sich bekannte Weise durch Umsetzung von Glyoxal, Formaldehyd und Ammoniak bei Temperaturen um 100 °C erhalten wurde und die ausser Wasser 9 Gew.-% Imidazol, 4 Gew.-% Ammoniumsalze organischer Säuren, 1 Gew.-% nicht umgesetztes Ammoniak und 2 Gew.-% Polymere und Kondensationsprodukte enthält, werden stündlich 300 Gew.-Teile über die Zuleitung 1 auf den Kopf einer pulsierenden Extraktionskolonne 2 gemäss Abbildung gegeben. Die Kolonne ist mit einer 4 m hohen Füllkörperschüttung versehen. Gleichzeitig werden über die Zuleitung 3 330 Gew.-Teile Pentanol eingeführt und bei Raumtemperatur im Gegenstrom zur Imidazollösung geleitet.

Die mit Imidazol angereicherte Lösungsmittelphase gelangt über den Kopf der Kolonne 4 in eine Rektifizierkolonne 5, in der sie durch Destillation in Pentanol und Imidazol zerlegt wird: Das Pentanol gelangt über die Leitung 6 wieder in die Extraktionskolonne. Das Imidazol wird der Destillationskolonne in Seitenabzug 7 entnommen; es hat eine Reinheit von 99,8% und eine Säurezahl von 0,3. Der Imidazol-Gehalt im wässrigen Raffinat, das der Kolonne über die Ableitung 8 entnommen wird, beträgt etwa 0,05 Gew.-%. Die Rückstände bei der Destillation der Lösungsmittelphase werden über die Ableitung 9 entfernt.

Vergleichsbeispiel

Verfährt man wie im Beispiel 1 beschrieben, wobei man jedoch anstelle von Pentanol Methylenchlorid verwendet, so erhält man eine Extraktphase, die nur ca. 4% des in der Ausgangslösung enthaltenen Imidazols enthält. Etwa 96% des Imidazols bleiben in der wässrigen Phase zurück.

Will man das gesamte Imidazol in die organische Phase überführen, so benötigt man bei einem stündlichen Zulauf von 300 Teilen Imidazollösung stündlich etwa 9000 Teile Methylenchlorid.

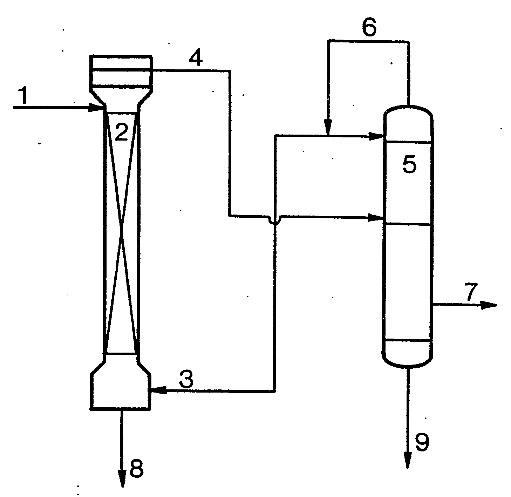

## Patentansprüche

1. Verfahren zur Gewinnung von Imidazolen aus ihren wässrigen Lösungen, die man durch Kondensation von Glyoxal mit Aldehyden und Ammoniak erhält, durch Extraktion mit einem Lösungsmittel und Destillation der Lösungsmittelphase, dadurch gekennzeichnet, dass man als Lösungsmittel mit Wasser nicht mischbare aliphatische Alkohole mit 4 bis 10 C-Atomen verwendet.

2. Verfahren nach Anspruch 1, dadurch gekennzeichnet, dass man als Lösungsmittel n-Butanol, iso-Butanol, Pentanol oder 2-Äthylhexanol verwendet.

3. Verfahren nach Anspruch 1, dadurch gekennzeichnet, dass man die Extraktion im Gegenstrom vornimmt.

## Claims

1. A process for isolating imidazoles from their aqueous solutions, obtained by condensing glyoxal with aldehydes and ammonia, by extracting with a solvent and distilling the solvent phase, wherein a water-immiscible aliphatic alcohol of 4 to 10 carbon atoms is used as the solvent.

2. A process as claimed in claim 1, wherein the solvent used is n-butanol, iso-butanol, pentanol or 2-ethylhexanol.

3. A process as claimed in claim 1, wherein the extraction is carried out in counter-current.

## Revendications

1. Procédé pour l'obtention d'imidazoles à partir de leurs solutions aqueuses, que l'on obtient par condensation de glyoxal avec des aldéhydes et l'ammoniac, par épuisement avec un solvant et distillation de la phase solvant, caractérisé en ce qu'on utilise, en tant que solvants, des alcools aliphatiques non miscibles à l'eau, comportant 4 à 10 atomes de carbone.

2. Procédé selon la revendication 1, caractérisé en ce qu'on utilise, en tant que solvant, le n-butanol, l'iso-butanol, le pentanol ou le 2-éthylhexanol.

3. Procédé selon la revendication 1, caractérisé en ce qu'on procède à l'extraction à contre-courant.